**Europäisches Patentamt**

**European Patent Office**

**Office européen des brevets**

(11) Publication number: **0 122 026**
**B1**

(12) ## EUROPEAN PATENT SPECIFICATION

(45) Date of publication of patent specification: **19.11.87**

(51) Int. Cl.⁴: **C 07 D 301/02, B 01 J 27/18**

(21) Application number: **84301518.1**

(22) Date of filing: **07.03.84**

(54) **Method for the manufacture of epoxides from 1,2-glycols.**

(30) Priority: **09.03.83 US 473837**

(43) Date of publication of application:
**17.10.84 Bulletin 84/42**

(45) Publication of the grant of the patent:
**19.11.87 Bulletin 87/47**

(84) Designated Contracting States:
**BE DE FR GB IT NL**

(56) References cited:
**DE-A-1 940 205**
**DE-A-2 812 439**
**FR-A-2 345 440**
**US-A-2 501 042**
**US-A-3 758 612**

(73) Proprietor: **ATLANTIC RICHFIELD COMPANY**
**515 South Flower Street**
**Los Angeles California 90071 (US)**

(72) Inventor: **Gastinger, Robert G.**
**4502 School Lane**
**Brookhaven, PA 19015 (US)**

(74) Representative: **Cropp, John Anthony David**
**et al**
**MATHYS & SQUIRE 10 Fleet Street**
**London, EC4Y 1AY (GB)**

# Description

This invention relates to the manufacture of epoxides.

More specifically, this invention relates to the manufacture of epoxides from 1,2 glycols.

In one of its more specific aspects, this invention pertains to a method for converting 1,2 propylene glycol directly to propylene oxide without forming an ester intermediate.

The vapor phase and liquid phase cracking of propylene glycol monoacetate ester to propylene oxide is well known. In general for this process feeds include various mixtures of propylene glycol mono- and di-acetate often containing some propylene glycol. However, an available source of ester, usually from the acetoxylation of propylene, is required or is generated in situ through, for example, the use of an alkali metal acetate supporting catalyst. U.S. 4,226,780 teaches the manufacture of propylene oxide from 1,2 propylene glycol using as the catalyst a weakly acidic carrier and an added basic alkali metal salt of a low molecular weight carboxylic acid.

The direct vapor phase dehydration of 1,2 glycols to epoxides, i.e. without the formation of an ester intermediate, has also been proposed. For example, FR—A—2345440 describes contacting the glycol in the vapor phase and at a temperature of 250 to 550°C, with a basic substance as catalyst. In one Example, which employs a supported sodium orthophosphate catalyst, a selectivity of 30.8% is reported. USP 2,501,042 reports a selectivity of only 8.6% when a catalyst based on $NaH_2PO_4$, $H_2O$ is employed.

According to this invention there is provided a method for the production of epoxides which comprises heating a 1,2 glycol in the vapor phase at a reaction temperature of from about 250 to 500°C over a catalyst which consists essentially of dibasic alkali metal phosphate supported or unsupported on a carrier.

In a preferred embodiment, the process of this invention is employed to provide propylene oxide directly from 1,2 propylene glycol.

In another embodiment, it has been found that the addition of acetic acid to the glycol feed significantly enhances epoxide selectivity without affecting catalyst activity.

The alkali metal may be, for example, lithium, sodium or, preferably, potassium. Carriers found useful are α-alumina, zircon and a refractory metal oxide consisting of 94% by weight α-alumina, 5% by weight CaO, and 1% CuO. The preferred carrier is α-alumina. In general for supported phosphates 5—20 weight percent of the salt on the carrier is employed.

In the practice of this invention, the 1,2 glycol feed concentrations, acetic acid feed concentrations and residence times based on catalyst void space can be varied between wide ranges. Preferably, 1,2 glycol feed concentrations will be within the range of 10—70 mol %. Acetic acid feed concentration, if employed, will be within the range of from about 0.5 to 5 mol %. Residence times will be within the range of from about 0.5 and 2 seconds.

Suitable phosphates are the dibasic phosphates i.e. having the formula $M_2HPO_4$.

The following examples further demonstrate catalyst preparation and the manufacture of epoxides according to this invention.

## Example I

This example demonstrates the preparation of a dipotassium hydrogen phosphate catalyst and its use to produce propylene oxide from propylene glycol.

About 5.0 grams of dipotassium hydrogen phosphate ($K_2HPO_4$) dissolved in about 22 ml of water were added to about 50.0 g of α-alumina. The resulting mixture was dried, air calcined at 425°C for about 10 hours and sieved to 8—14 mesh.

A stainless steel tubular reactor about 14.3 mm (9/16″) internal diameter was packed with 30 ml of the potassium phosphate catalyst.

At 401°C a feed stream of 38 mol % propylene glycol in nitrogen was vaporized and passed over the catalyst at a rate of 29.0 g propylene glycol per hour.

The products were collected and analyzed by gas chromatography. The conversion of propylene glycol was 19% with a 70% propylene oxide and 19% propanal selectivity. The remaining products consisted primarily of acetone, allyl alcohol, 1-propanol, propylene and acetol (hyroxyacetone).

## Example II

This example further demonstrates the use of the catalyst produced in Example I to produce propylene oxide from propylene glycol. Acetic acid was employed in the feed stream to improve propylene oxide selectivity.

A stainless steel tubular reactor about 14.3 mm (9/16″) internal diameter was packed with 30 ml of the catalyst.

At 398°C a feed stream of 30 mol % propylene glycol and 1.4 mol % acetic acid in nitrogen was vaporized and passed over the catalyst at a rate of 18.5 g propylene glycol per hour.

The products were collected and analyzed by gas chromatography. The conversion of propylene glycol was 25% with a 77% propylene oxide and 10% propanal selectivity. The remaining products consisted primarily of acetone, allyl alcohol, 1-propanol, propylene and acetol (hydroxyacetone).

# Claims

1. A method for the production of an epoxide by heating a 1,2 glycol stream in the vapor phase at a reaction temperature of from 250 to 500°C and in the presence of an alkali metal compound as catalyst characterised in that the catalyst consists essentially of a dibasic alkali metal phosphate supported or unsupported on a carrier.

2. A method as claimed in claim 1 characterised in that said alkali metal is potassium.

3. A method as claimed in claim 1 or claim 2 characterised in that an effective amount of acetic acid is added to the 1,2 glycol stream to improve selectivity.

4. A method as claimed in any one of claims 1 to 3 in which propylene oxide is formed from a 1,2 propylene glycol stream.

5. A method as claimed in any one of claims 1 to 4 caracterised in that the catalyst is supported on α-alumina.

## Patentansprüche

1. Verfahren zur Herstellung eines Epoxids durch Erhitzen eines 1,2-Glykolstroms in der Dampfphase bei einer Reaktionstemperatur von 250 bis 500°C und in Gegenwart einer Alkalimetallverbindung als Katalysator, dadurch gekennzeichnet, daß der Katalysator im wesentlichen aus einem dibasischen Alkalimetallphosphat, gestützt oder ungestützt durch einen Träger, besteht.

2. Verfahren nach Anspruch 1, dadurch gekennzeichnet, daß das Alkalimetall Kalium ist.

3. Verfahren nach Anspruch 1 oder 2 dadurch gekennzeichnet, daß eine wirksame Menge Essigsäure zum 1,2-Glykolstrom zur Verbesserung der Selektivität zugegeben wird.

4. Verfahren nach einem der Ansprüche 1 bis 3, worin Propylenoxid aus einem 1,2-Propylen-Glykolstrom gebildet wird.

5. Verfahren nach einem der Ansprüche 1 bis 4, dadurch gekennzeichnet, daß der Träger für den Katalysator alpha-Aluminium ist.

## Revendications

1. Procédé de préparation d'un époxyde par chauffage d'un courant de 1,2-glycol en phase vapeur à une température de réaction de 250 à 500°C et en présence d'un composé de métal alcalin comme catalyseur, caractérisé en ce que le catalyseur consiste essentiellement en un phosphate dibasique de métal alcalin supporté ou non sur un support.

2. Procédé suivant la revendication 1, caractérisé en ce que ce métal alcalin est le potassium.

3. Procédé suivant la revendication 1 ou 2, caractérisé en ce qu'une quantité efficace d'acide acétique est ajoutée au courant de 1,2-glycol pour améliorer la sélectivité.

4. Procédé suivant l'une quelconque des revendications 1 à 3, dans lequel l'oxyde de propylène est formé à partir d'un courant de 1,2-propylèneglycol.

5. Procédé suivant l'une quelconque des revendications 1 à 4, caractérisé en ce que le catalyseur est supporté sur de l'α-alumine.